(19) Europäisches Patentamt
European Patent Office
Office européen des brevets

(11) **EP 1 183 511 B1**

(12) **EUROPÄISCHE PATENTSCHRIFT**

(45) Veröffentlichungstag und Bekanntmachung des Hinweises auf die Patenterteilung:
**13.08.2003 Patentblatt 2003/33**

(51) Int Cl.⁷: **G01L 19/04**

(86) Internationale Anmeldenummer:
**PCT/CH00/00281**

(21) Anmeldenummer: **00925013.5**

(22) Anmeldetag: **19.05.2000**

(87) Internationale Veröffentlichungsnummer:
**WO 00/071982 (30.11.2000 Gazette 2000/48)**

(54) **DRUCKMESSVORRICHTUNG**

PRESSURE-MEASURING DEVICE

DISPOSITIF DE MESURE DE PRESSION

(84) Benannte Vertragsstaaten:
**CH DE FR GB LI**

(30) Priorität: **19.05.1999 CH 94299**

(43) Veröffentlichungstag der Anmeldung:
**06.03.2002 Patentblatt 2002/10**

(73) Patentinhaber: **SMT Swiss Microtechnology AG**
**2562 Port (CH)**

(72) Erfinder: **KANNGIESSER, Hartmut**
**CH-8038 Zürich (CH)**

(74) Vertreter:
**Roshardt, Werner Alfred, Dipl.-Phys. et al**
**Keller & Partner**
**Patentanwälte AG**
**Schmiedenplatz 5**
**Postfach**
**3000 Bern 7 (CH)**

(56) Entgegenhaltungen:
**US-A- 3 990 309          US-A- 3 999 435**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft eine Druckmessvorrichtung zur Messung des Augendrucks.

## Stand der Technik

Bekannte Druckmessvorrichtungen mit einer Flüssigkeit als Druckübertragungsmittel zu einem Druckmesssensor hatten ein Volumenausgleichselement, mit dem eine Volumenvergrösserung oder -Verkleinerung der Flüssigkeit infolge einer Temperaturerhöhung oder - erniedrigung ausgeglichen werden konnte. Das Volumenausgleichselement war beispielsweise eine mit der Flüssigkeit in Verbindung stehende Kolben-Zylinder-Einheit, bei welcher der Kolben entsprechend der Temperaturänderung bewegt wurde. Druckübertragungsmittel, insbesondere -flüssigkeiten werden überall dort eingesetzt, wo ein zu messender Druck nicht direkt auf einen Messsensor aufgebracht werden kann.

In der US-A 3 990 309 ist eine Druckmessvorrichtung mit einer Temperaturkompensation beschrieben. Zur Temperaturkompensation wird ein Edelstahlgehäuse mit einem innenliegenden Klotz aus invar verwendet. Als Druckübertragungsmittel wird ein Öl verwendet, welches den Invarklotz umgibt und den Zwischenraum zwischen Klotz und der Innenwand des Edelstahlgehäuses ausfüllt. Zur Druckbestimmung wird dieses Öl aus dem Zwischenraum in eine sogenannte Bourdon-Röhre geführt, an deren Röhrenende ein durch eine Kristallplatte sichtbarer Zeiger zur Druckanzeige angeordnet ist.

Die aus der US-A 3 990 309 bekannte temperaturkompensierte Druckmesseinrichtung wird in der Luftfahrt bei Drücken bis zu 207 bar (3000 psi) und grossen Temperaturunterschieden (-51°C bis 93°C) (-60°F bis + 200°F) eingesetzt.

## Aufgabe der Erfindung

Aufgabe der Erfindung ist es, eine Druckmessvorrichtung mit einem fliessfähigen Druckübertragungsmittel mit vereinfachter Bedienung zu schaffen.

## Lösung der Aufgabe

Unter einem fliessfähigen Druckübertragungsmittel werden bevorzugt Flüssigkeiten verstanden; es können aber auch gallertartige Massen, insbesondere Gelee eingesetzt werden. Es hatte sich gezeigt, dass eine Volumenverstellung des Druckübertragungsmittels zum Ausgleich einer Volumenänderung infolge von Temperaturänderungen aufwendig war und zudem oftmals Fehlmessungen nach sich zog. Die Erfindung schlägt nun einen anderen Weg ein, indem sie auf eine einstellbare Volumenveränderung verzichtet. Statt einer einstellbaren Volumenveränderung bei einer Temperaturänderung schlägt die Erfindung vor, Materialien und Abmessungen der Hohlraumbegrenzungen und der Drückmesseinheit sowie die Art des Druckübertragungsmittels derart zu wählen, dass über einen vorgegebenen Temperaturbereich ohne äussere Einwirkung der Flächendruck auf die Begrenzungswände des Druckübertragungsmittels ohne äussere Druckeinwirkung konstant bleibt. In einer bevorzugten Ausführungsvariante der Erfindung ist in das Druckübertragungsmittel ein Ausgleichskörper eingebracht.

Die temperaturbedingte Volumenänderung der Druckübertragungsflüssigkeit wird nun erfindungsgemäss durch ein thermisches Zusammenspiel von thermischer Volumenänderung, insbesondere unter Einwirkung des Ausgleichskörpers und des das Druckübertragungsmittel aufnehmenden Hohlraums unter einer nahzu vernachlässigbaren Berücksichtigung der thermischen Ausdehnung der Druckmesseinheit ausgeglichen.

Erfindungsgemäss sind Druckübertragungsmittel sowie Hohlraumwände und wenigstens Teilbereiche der Druckmessvorrichtung optisch transparent ausgebildet, wodurch die Druckmessvorrichtung in ein optisches Betrachtungssystem z.B. bei der Augendruckmessung integrierbar ist. Das verwendete Druckübertragungsmittel ist dann auch mit entsprechendem optischen Brechungsindex ausgewählt. Im der Ansgestaltung als Augendruckmessvorrichtung ist in einer der Hohlraumwände eine Druckübertragungsmembran angeordnet, welche mit der Druckübertragungsflüssigkeit in Verbindung steht. Die Membran ist ebenfalls in einem Wellenlängenbereich des sichtbaren Lichts transparent ausgebildet.

Weitere Vorteile der Erfindung sowie der Ausführungsvarianten ergeben sich aus dem untenstehenden Text.

## Kurze Beschreibung der Zeichnungen

Im folgenden werden Beispiele der erfindungsgemässen Druckmeßvorrichtung anhand der Zeichnungen näher erläutert. Es zeigen:

Fig. 1    einen Längsschnitt durch die als Kontaktglas ausgebildete Druckmeßvorrichtung,

Fig. 2    eine skizzenhafte Darstellung einer in der in **Figur 1** dargestellten Druckmeßeinheit in größerem Maßstab und

Fig. 3    einen Querschnitt durch die Einbaustelle der Druckmeßeinheit in einen Grundkörper der in **Figur 1** dargestellten Druckmeßvorrichtung.

**Wege zur Ausführung der Erfindung**

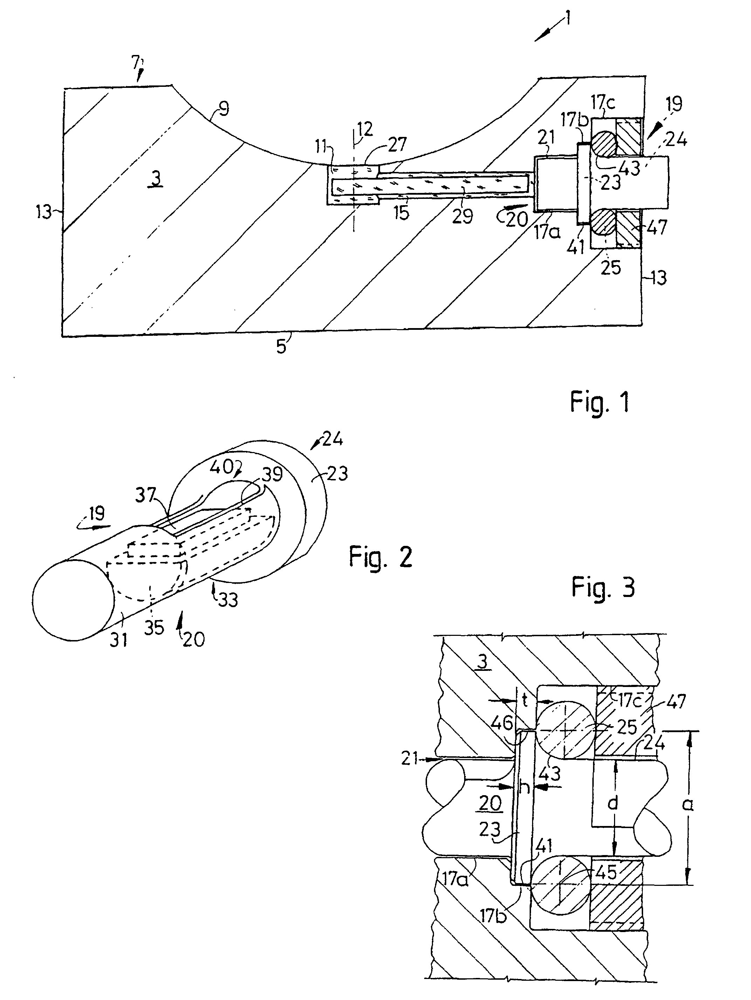

Die in **Figur 1** dargestellte Druckmeßvorrichtung **1** ist als Kontaktglas zur Augendruckmessung ausgebildet. Die Druckmeßvorrichtung **1** hat hier beispielsweise einen zylindrischen Grundkörper **3** mit einer ebenen, optisch planen Grundfläche **5**, der benachbart ein nicht dargestelltes optisches Beobachtungssystem sowie eine nicht dargestellte Beleuchtungseinheit angeordnet ist. Die der Grundfläche **5** gegenüberliegende Seite **7** hat eine konkave Ausnehmung **9**, deren Flächenkontur der menschlichen Augenwölbung angepaßt ist und hier ebenfalls eine optische Oberflächenqualität hat. im Scheitelpunkt der Ausnehmung **9** ist eine Zylindersacklochbohrung **11** vorhanden. In diese Sacklochbohrung **11** mündet senkrecht zu deren Mittellinie **12** eine weitere, senkrecht zur Mantelfläche **13** des Grundkörpers **3** verlaufende Zylinderbohrung **15**. Diese Zylinderbohrung **15** ist zentrisch mit einer sich dreistufig zur Mantelfläche **13** erweiternden, weiteren Zylinderbohrung **17** verbunden. In die durch die dreistufige Formgebung gebildeten Teilbohrungen **17a, 17b** und **17c** ist eine Druckmeßeinheit **19** eingesetzt. Der Meßkopf **20** und der Absatz **23** der Druckmeßeinheit **19** ragen, umgeben von umlaufenden Spalten **21** und **41,** in die Teilbohrungen **17a** und **17b** hinein. In der Teilbohrung **17c** ist um einen Schaft **24** der Druckmeßeinheit **19** ein Dichtring **25** gelegt, der mit einer in die Teilbohrung **17c** einschraubbaren Mutter **47** flüssigkeitsdicht einpreßbar ist.
Die Sackbohrung **11** ist nach außen mit einer, hier beispielsweise optisch transparenten Membran **27** abgeschlossen. In der Zylinderbohrung **15** liegt ein Ausgleichskörper **29**. Die Sackbohrung **11,** die Zylinderbohrung **15,** der Spalt **21** in der Teilbohrung **17a** sowie der Spalt **41** in der Teilbohrung **17b** bilden einen gemeinsamen Hohlraum, der mit einer Flüssigkeit als Druckübertragungsmittel gefüllt ist.
Das Material des Grundkörpers **3** ist somit das Material der Begrenzung des gemeinsamen Hohlraums, in dem die Flüssigkeit eingeschlossen ist. Das Material des Grundkörpers **3,** des Ausgleichskörpers **29** und des Meßkopfes **20** sowie deren Abmessungen und die Art der Flüssigkeit sind nun derart gewählt, daß über einen vorgegebenen Temperaturbereich ohne äußere Einwirkung der Flüssigkeitsdruck konstant bleibt. D.h. der Druckmeßkopf **20** mißt ohne äußere Druckbeaufschlagung bei einer Temperaturänderung im vorgegebenen Temperaturbereich auch keinen sich ändernden Druck. Ein Druck auf die Membran **27** wird demzufolge ohne Temperaturbeeinflussung auf den Druckmeßkopf **20** übertragen.
Zum besseren Verständnis der Erfindung wird zuerst lediglich ein mit einer beliebigen Flüssigkeit gefüllter Hohlraum ohne Ausgleichskörper **29** zur Druckübertragung betrachtet.
Die Druckübertragung von der Membran **27** bis zum Meßkopf **20** erfolgt über den flüssigkeitsgefüllten Hohlraum. Die Wände des die Flüssigkeit aufnehmenden Hohlraums sowie auch die Flüssigkeit sind einer thermischen Ausdehnung unterworfen. Sind die Ausdehnungen unterschiedlich, so entstehen bei einem abgeschlossenen System temperaturbedingte Druckänderungen, welche den mit der Membran **27** aufzunehmenden Druck verfälschen würden. Man kann nun die Druckübertragungsflüssigkeit und das die Flüssigkeit umgebende Material derart aufeinander abstimmen, daß eine Temperaturkompensation gegeben ist. Es tritt somit eine Überlagerung der thermischen Hohlraumvergrößerung und der thermischen Ausdehnung der Flüssigkeit auf. Wird als Hohlraumwandung ein Material mit relativ hohem thermischen Ausdehnungskoeffizienten verwendet (z.B. ein Kunststoff), so kann bei spezieller Auswahl eine dem thermischen Verhalten des Hohlraums angepaßte Flüssigkeit mit einem niedrigen Ausdehnungskoeffizienten, wie beispielsweise Wasser, gefunden werden. Es treten hierbei jedoch Schwierigkeiten auf, da in den allgemein zu verwendenden Temperaturbereichen (20°C bis 40°C) derartige Flüssigkeiten in ihrem Ausdehnungsverhalten starke Nichtlinearitäten aufweisen. Eine allgemein gültige Anpassung ist hier demzufolge nicht erreichbar.
Um die eben genannten Schwierigkeiten zu umgehen, wird in einer Ausführungsvariante der Erfindung, wie sie die **Figur 1** zeigt, ein Ausgleichskörper **29** verwendet. Feststoffe haben im Allgemeinen einen niedrigeren Ausdehnungskoeffizienten als Flüssigkeiten. Es existieren weiterhin Feststoffe, wie beispielsweise Gläser und bestimmte Metalle, welche sehr niedrige Ausdehnungskoeffizienten aufweisen. Werden nun Gegenstände derartiger Feststoffe als Ausgleichskörper zusammen mit einer druckübertragenden Flüssigkeit in den die Flüssigkeit aufnehmenden Hohlraum eingebracht, so ist eine Kompensation möglich.
Durch den Ausgleichskörper **29** in der Druckübertragungsflüssigkeit ergibt sich für das gesamte Druckübertragungsmittel (Flüssigkeit + Körper) ein resultierender Ausdehnungskoeffizient, wodurch bei geeigneter Materialwahl des Ausgleichskörpers auf eine Flüssigkeit mit sehr niedrigem Ausdehnungskoeffizienten mit den bekannten negativen Eigenschaften verzichtet werden kann. Es ist jedoch darauf zu achten, daß ein verbleibender Druckübertragungsmittelspalt zwischen Ausgleichskörper und den Hohlraumwänden noch immer so groß bleibt, daß nicht infolge von Kapillarwirkung die Druckübertragung von der Druck aufnehmenden Membran **27** zur Druckmeßeinheit **19** beeinträchtigt wird.

Nachfolgend wird ein Beispiel einer Druckmeßvorrichtung beschrieben, welche zur Augendruckmessung verwendbar ist. D.h. die hier beschriebene Druckmeßvorrichtung erfüllt neben einer Temperaturkompensation bei der Druckübertragung zusätzlich die Bedingung, daß sie optisch transparent ist.

Das Material des Grundkörpers **3**, hier ein sog. Linsenkörper, ist Plexiglas. Als Flüssigkeit wird ein Siliconölgemisch, wie unten beschrieben, verwendet. Der Ausgleichskörper ist aus Glas. Die innerhalb der Druckübertragungsstrecke befindlichen Materialien ausgehend von der durchsichtigen Membran **27** über die Sackbohrung **11**, die Bohrung **15**, der Glasstab **29** und das Siliconölgemisch weisen einen resultierenden Ausdehnungskoeffizienten auf, der gleich demjenigen des Plexiglases ist. Der Meßkopf **20** der Druckmeßeinheit **19** ragt in die Zylinderbohrungen **17a** und **17b** hinein und ist hier von Siliconöl umschlossen. Auch hier gelten dieselben Betrachtungen wie in der Druckübertragungsstrecke. Der sich aus dem Meßkopf **20** und aus der umgebenden Flüssigkeit (Siliconölgemisch) ergebende weitere resultierende Ausdehnungskoeffizient muß ebenfalls gleich demjenigen des Plexiglases sein. Der Meßkopf **20** setzt sich aus verschiedenen Materialien zusammen, so daß sich sein thermisches Ausdehnungsverhalten aus einem Zusammenspiel unterschiedlicher Ausdehnungskoeffizienten ergibt.

Bei der Konstruktion dieser Druckmeßvorrichtung wird bevorzugt darauf geachtet, daß möglichst an jeder Stelle der zur Druckübertragung dienende Hohlraum (**11** und **15**) sich gerade so stark ausdehnt, wie die darin sich befindende Werkstoffkombination. Diese Verhältnisse sollten an jeder Stelle erreicht werden und nicht nur summarisch über den gesamten Weg. Im letzteren Fall würde nämlich ein vorhandener Temperaturgradient zu einer Fehlkompensation führen. Derartige Temperaturgradienten könnten insbesondere bei einer Anwendung der Druckmeßvorrichtung am körperwarmen Auge entstehen.

Die Druckmeßvorrichtung wird bei Normaldruck eingesetzt. Der zu messende Druck liegt nur geringfügig über Normaldruck. Es kann also davon ausgegangen werden, daß sämtliche Festkörper im Druckmeßbereich inkompressibel sind. Auch die verwendete Flüssigkeit wird als inkompressibel betrachtet. Um die Flüssigkeit inkompressibel zu erhalten, muß darauf geachtet werden, daß sie gasfrei ist. Zur Vermeidung der oben erwähnten Fehlmessung infolge von auftretenden Temperaturgradienten ist der gesamte Hohlraum, bestehend im wesentlichen aus den beiden Bohrungen **11** und **15**, an nahezu jeder Stelle mit dem Ausgleichskörper **29** bestückt.

Das thermische Verhalten der Druckmeßeinheit **19** ist nur soweit zu betrachten, wie deren Teile mit der Druckübertragungsflüssigkeit in Kontakt sind.; d.h. alle Teile, welche in **Figur 1** links vom Dichtring **25** liegen. Eine beispielsweise Ausgestaltung der Druckmeßeinheit **19** zeigt **Figur 2**. Der Meßkopf **20** sitzt auf dem zylindrischen Absatz **23.** Der zylindrische Absatz **23** und ein vorderer Teil **31** des Meßkopfes **20** bestehen aus einer Stahlhülse, in die ein Kern aus Keramik eingeklebt ist. Deren thermisches Verhalten läßt sich nur schwer berechnen, da die Stahlhülse das Ausdehnungsverhalten des Keramikkerns beeinflußt und umgekehrt. In einem Bereich **33** zwischen dem Absatz **23** und dem Teil **31** sind alle Materialien so angeordnet, daß sie sich frei ausdehnen können. Es handelt sich um eine nach einer Seite offene Hülse **34** aus Stahl, ein darin eingeklebtes Keramikteil **35** mit etwa halbkreisförmigem Querschnitt sowie ein auf den Keramikteil aufgeklebtes Sensorelement **37** aus Silizium. Der verbleibende Raum um das Sensorelement **37** herum und darüber bis zur Oberkante **39** der Öffnung **40** in der Stahlhülse **34** ist mit einem flexiblen Silicon ausgegossen.

Der Sensor ist somit aus unterschiedlichen Materialien A, B, C, ... aufgebaut, die unterschiedliche thermische Ausdehnungskoeffizienten $\alpha_A$, $\alpha_B$, $\alpha_C$, ... und unterschiedliche Volumina $\mathbf{V}_A$, $\mathbf{V}_B$, $\mathbf{V}_C$ aufweisen. Es ist zu beachten, daß die thermischen Ausdehnungskoeffizienten durch andere Materialien in der Umgebung des entsprechenden Bauteils bzw. Materials beeinflußt werden können (keine freie Ausdehnung). Ein resultierender thermischer Ausdehnungskoeffizient $\alpha_{r20,23}$ des Meßkopfes **20** und des Absatzes **23** ergibt sich dann zu

$$\alpha_{r20,23} = (\alpha_A \cdot V_A, + \alpha_B \cdot V_B + \alpha_C \cdot V_C + ...) / (V_A + V_B + V_C + ...).$$

Der resultierende Ausdehnungskoeffizient kann gemäß vorstehender Formel berechnet werden. In der Regel wird man ihn aber bei zusammengesetzten Bauteilen experimentell (beispielsweise durch Erwärmen in einer Flüssigkeit mit bekanntem thermischem Ausdehnungskoeffizient) ermitteln. Das Volumen der herzustellenden Bohrungen **17a** und **17b** wird nun aus der nachfolgenden Beziehung ermittelt

$$\alpha_{Plexi} \cdot V_{17a,\,17b} = \alpha_{Öl} \cdot V_{21,41} + \alpha_{r20,\,23} \cdot V_{20,\,23},$$

wobei

$\alpha_{\mathbf{Plexi}}$ der thermische Ausdehnungskoeffizient des die Wände der Bohrungen **17a** und **17b** bildenden Materials, hier des Materials des Grundkörpers **3** Plexiglas, ist;

$V_{17a, 17b}$ ist das Volumen der Bohrungen **17a** und **17b;**

$\alpha_{Öl}$ ist der thermische Ausdehnungskoeffizient des Siliconölgemisches;

$V_{21,41}$ ist das Volumen der Spalte **21** und **41** in den Bohrungen **17a** und **17b** zwischen der Bohrungswandung und dem Meßkopf **20** bzw. dem Absatz **23;**

$V_{20,23}$ ist das Volumen des Meßkopfes **20** und des Absatzes **23.**

Aus dem Volumen $V_{17a, 17b}$ kann der Durchmesser und die Länge der Bohrungen **17a** und **17b** in einer sich zur Herstellung besonders eignenden Form ermittelt werden. Ein zu vermeidender thermischer Temperaturgradient tritt in den Bohrungen **17a** und **17b** so gut wie nicht auf, da der Meßkopf **20** und der Absatz **23** eine gute thermische Wärmeleitfähigkeit (deren Material ist zum größten Teil Stahl) haben.

In den Bohrungen **11** und **15** ist zur thermischen Kompensation ein Glaskörper **29** eingebracht. Es gilt dort somit analog in der Bohrung **15**

$$\alpha_{Plexi} \cdot V_{15} = \alpha_{Öl} \cdot V_{S15} + \alpha_{Glas} \cdot V_{Glas\ 15},$$

sowie in der Bohrung **11** :

$$\alpha_{Plexi} \cdot V_{11} = \alpha_{Öl} \cdot V_{S11} + \alpha_{Glas} \cdot V_{Glas\ 11},$$

wobei $V_{S15}$ und $V_{S11}$ die Volumendifferenz zwischen Glasstab und dem jeweilgen Bohrlochvolumen angibt. In den Bohrungen **17a** und **17b** dient der Meßkopf **20** bzw. der Absatz **23** als Ausgleichskörper.

Für die benötigte Volumenmenge $V_{29}$ Glas, d.h. für das Volumen des Ausgleichskörpers **29** ergibt sich dann

$$V_{29} = \frac{\alpha_{Öl} - \alpha_{Plexi}}{\alpha_{Plexi} - \alpha_{Glas}} \cdot V_{Öl}$$

Werden nicht allzu hohe Anforderungen an die Temperaturkompensation gestellt, kann auf einen Ausgleichskörper in der Bohrung **11**, welche mit der Membran abgeschlossen ist, verzichtet werden.

Anstelle der oben angeführten Materialien können auch andere Materialien verwendet werden. So kann beispielsweise bei einer optisch nicht durchsichtigen Druckmeßvorrichtung mit einem metallenen Ausgleichskörper z.B. aus Stahl gearbeitet werden. Anstelle eines einzigen Ausgleichskörpers können auch mehrere verwendet werden.

Ein Spalt **41** zwischen dem Absatz **23** und der Wandung der Bohrung **17b** ist, wie Figur **3** zeigt, gerade so eng gemacht, daß beim Einstecken des Meßkopfes noch Flüssigkeit nach außen dringen kann, damit beim Einschieben kein Druckaufbau in den Bohrungen **15** und **11** erfolgen kann. Die Bohrung **17c** ist größer als der Außendurchmesser des Dichtrings **25**. Der Durchmesser der Bohrung **17b** ist um eine Toleranz größer als der Mittendurchmesser des Dichtrings **25**. Der Dichtring **25** sitzt bündig auf dem Schaft **24** der Druckmeßeinheit **19**. Ein Übergang zwischen der radialen Außenkante **46** des Absatzes **23** und dem Schaft **24** ist als viertelkreisförmige Kontur **43** ausgebildet und damit in diesem Bereich der Außenkontur des Dichtrings **25** angepaßt.

Die Höhe **h** des Absatzes **23** und die Tiefe **t** der Teilbohrung **17b** sind gleich groß gewählt. Femer ist der Außendurchmesser **a** des Absatzes **23** so groß ausgebildet wie der Durchmesser der Querschnittsmitte **45** des Dichtrings **25**. Der Durchmesser **d** des Schafts **24** entspricht dem Innendurchmesser des Dichtrings **25**. Der Durchmesser der Teilbohrung **17c** ist so gewählt, daß der Dichtring **25** mit Spiel einsetzbar ist.

Die Druckmeßeinheit **19** wird flüssigkeitsdicht eingesetzt, indem eine Verschlußmutter **47** als Verschlußmittel in die als Verschlußbuchse ausgebildete Teilbohrung **17c** eingeschraubt wird. Da die Dichtringquerschnittsmitte **45** über der radialen Begrenzung des Absatzes **23** liegt und der Dichtring **23** durch elastische Verformung sich nur in radialer Richtung über den Spalt **41** schieben kann, wird beim Vorgang des Verschliessens mit dem Dichtring **25** die überschüssige Flüssigkeit durch seine Verformung radial nach außen abgeschert. Da die Spaltbreite des Spaltes **41** gegenüber dem Querschnitt des Dichtrings **25** verschwindend ist, wird dieser nur unwesentlich in den Spalt **41** hineingedrückt. Ein Druckaufbau der Druckübertragungsflüssigkeit wird hierdurch auf ein Minimum begrenzt.

Die Bohrung **17b** und der Absatz **23** müssen nicht als gerade Kreiszylinder ausgebildet werden. Es sind auch andere Formen wie beispielsweise konische Formen möglich. Die Querschnittsmitte des Dichtrings **25** sollte jedoch immer möglichst genau über der radialen Außenkante, welche in diesem Fall nicht mehr parallel zur Achse verläuft, liegen. Auch sollte der Spalt zwischen Bohrung und Absatz schmal gewählt werden.

Die als Druckübertragungsflüssigkeit zu verwendende Flüssigkeit muß neben guten optischen Eigenschaften noch die nachfolgenden Bedingungen erfüllen. Sie muß lichtecht (insbesondere UV-stabil) sein, eine sehr geringe Löslichkeit für Gase und Wasser haben und vakuumfest sein; d.h. nicht ausgasen. Ferner muß sie, falls sie in einer Druckmeßvorrichtung für den Augendruck verwendet wird, biologisch verträglich sowie chemisch mit allen verwendeten Reinigungsmitteln und den Materialien des Linsenkörpers (Grundkörper) **3,** des Glasstabs **29** und der Druckmeßeinheit **19** verträglich sein. Die Drucküberträgungsflüssigkeit darf insbesondere keine ihr Volumen und ihre optischen Eigenschaften verändernden Stoffe, seien sie flüssig, gasförmig oder fest, aufnehmen. Die Drucküberträgungsflüssigkeit darf keine Stoffe (Materialien) aus ihren Begrenzungen aufnehmen bzw. herauslösen. Sie darf auch keine Materialien aufnehmen, welche durch die Begrenzungen, aus der Umgebung stammend, hindurch diffundieren könnten.

Als Druckübertragungsflüssigkeiten können beispielsweise Öl, wie Siliconöle, naturliche Öle (Zedernholzöl, Leinöl, ...), auch Glyzerin, ... verwendet werden. Es können auch zur Anpassung des Brechungsindexes Mischungen von unterschiedlichen Ölen bzw. anderen transparenten Flüssigkeiten mit den oben genannten Bedingungen verwendet werden. Gute Ergebnisse wurden beispielsweise mit einer Mischung zweier Siliconöle der Firma Wacker-Chemie mit der Bezeichnung AP 100 und AR 20 erreicht. Die Mischung der beiden Öle ist auch noch unter dem Gesichtspunkt der Erzeugung eines Brechungsindexes, der demjenigen von Plexiglas möglichst nahe kommt, vorgenommen worden. Die hier beschriebene Druckmeßvorrichtung soll nämlich in ein optisches Betrachtungssystem integrierbar sein. Als Material für den Glasstab ist FK5 (Firma Schott) gewählt worden.

Die vorstehend aufgeführten Druckübertragungsflüssigkeiten stellen nur eine kleine Auswahl möglicher Materialien und deren Mischungen dar.

Eine exakte Temperaturkompensation wird erreicht, wenn gemäß oben stehendem Berechnungsbeispiel nicht auf Teilvolumen, sondern auf Flächenausdehnung in den drei Koordinatenrichtungen (karthesisch, Kugelkoordinaten) abgestellt wird. Eine gute Näherung hierzu bietet jedoch eine Kompensation in Teilvoluminas, sofern diese so gewählt sind, daß sich ein in ihnen ausbildender Temperaturgradient immer klein ist.

Die in **Figur 1** dargestellte Druckmeßvorrichtung **1** ist bis auf die Druckmeßeinheit **19** optisch transparent. Hierdurch ist eine einfache Herstellung möglich, da eine Beschränkung auf nur wenige Materialien erfolgt ist. Für die Verwendung der Druckmeßvorrichtung in der Ophthalmologie wäre jedoch ausreichend, wenn lediglich Bereiche der Druckmessvorrichtung, welche vom Beobachtungs- und/oder Beleuchtungsstrahlengang zu durchdringen sind, optisch transparent ausgebildet sind.

Wie oben ausgeführt mündet die Sacklochbohrung **11** senkrecht zu deren Mittellinie **12** eine weitere, senkrecht zur Mantelfläche **13** des Grundkörpers **3** verlaufende Zylinderbohrung **15**. Anstelle eines senkrechten Einmündens können selbstverständlich auch andere Einmündungswinkel vorgesehen werden.

**Patentansprüche**

1. Druckmessvorrichtung **(1)** zur Messung des Augendrucks mit einem einen Ausgleichs-Rörper **(29)** umgebenden Druckübertragungsmittel gefüllten Hohlraum **(11, 15, 17a, 17b),** der von Hohlraumwänden umgeben ist, und mit einer Druckmesseinheit **(19),** welche über das Druckübertragungsmittel mit einer Druckübertragungsmembran **(27)** in Verbindung steht, wobei die Materialien und Abmessungen der Hohlraumwände, des Hohlraumvolumens ($V_{11}$, $V_{15}$, $V_{17a}$, $V_{17b}$) und der Druckmesseinheit **(19)** sowie die Art des Druckübertragungsmittels derart gewählt sind, dass über einen vorgegebenen Temperaturbereich ohne äussere Einwirkung der Druck des Druckühertragungsmittels auf seine Begrenzungswände konstant bleibt und in wenigstens einer der Wände eines Hohlraums **(11)** die Druckübertragungsmembran **(27)** angeordnet ist, die Hohlraumwände, das Druckübertragungsmittel, die Druckübertragungsmembran **(27)**, der Ausgleichskörper **(29)** und der Grundkörper **(3)** der Vorrichtung in wenigstens einem Wellenlängenbereich des sichtbaren Lichts transparent sind und der Brechungsindex des Druckübertragungsmittels und Teilbereiche des Grundkörpers **(3)** und/oder des Ausgleichskörpers **(29)** einander angepasst sind, damit Betrachtungen durch die Vorrichtung hindurch mit einem optischen Beobachtungssystem am bzw. im Auge vornehmbar sind.

2. Druckmessvorrichtung **(1)** nach Anspruch 1, mit Aussenkonturen **(5, 9)** in wenigstens zwei einander gegenüberliegenden Teilbereichen der Vorrichtung **(1),** welche derart geformt sind, dass die Vorrichtung **(1)** in ein abbildendes optisches System einsetzbar ist.

3. Druckmessvorrichtung **(1)** nach Anspruch 1 oder 2, mit einer Flüssigkeit als Druckübertragungsmittel.

4. Druckmessvorrichtung nach Anspruch 3, bei der möglichst an jeder Stelle der zur Druckübertragung dienende Hohlraum sich gerade so stark ausdehnt, wie die darin sich befindende Werkstoffkombination aus Druckübertragungsmitteil und Ausgleichskörper, damit möglichst bei der Druckübertragung zwischen Druckübertragungsmembran **(27)** und Druckmesseinheit **(19)** an jedem Ort eine, und nicht nur eine summarische Kompensation erreicht wird, damit ein vorhandener Temperaturgradient zu keiner Fehlkompensation führt

**5.** Druckmessvorrichtung **(1)** nach einem der Ansprüche 1 bis 4, bei der das Druckübertragungsmittel gasfrei sowie bevorzugt inert ist gegenüber Materialien der Hohlraumwände bzw. Materialien aus diesen und/oder gegen durch diese hindurch diffundierende Materialien.

**6.** Druckmessvorrichtung **(1)** nach einem der Ansprüche 1 bis 5, bei der die Druckmesseinheit **(19)** einen Messkopf **(20)** und einen gegenüber dem Messkopfaussendurchmesser querschnittserweiterten Absatz **(23)** hat und eine der Hohlraumwände einen Durchbruch **(17a, 17b, 17c)** aufweist, dessen Abmessungen derart gewählt sind, dass der Messkopf **(20)** hindurchsteckbar ist und ein Teildurchbruch **(17b)** vorhanden ist, dessen Querschnitt zum Absatz **(23)** einen seitlichen Spalt **(41)** zulässt, die Teildurchbruchstiefe **(t)** sowie die Absatzhöhe **(h)** gleich gross gewählt sind und der Teildurchbruch **(17b)** sich auf seiner dem Hohlraum abgewandten Seite derart in einen Verschlussbuchsenbereich **(17c)** erweitert, dass ein Dichtmittel, insbesondere ein Dichtring **(25)** mit Spiel einsetzbar ist.

**7.** Druckmessvorrichtung **(1)** nach Anspruch 6, bei der an den Absatz **(23)** der Druckmesseinheit **(19)** ein im Durchmesser verringerter Schaft **(24)** anschliesst, und der Konturenübergang **(43)** vom Aussendurchmesser des Absatzes **(23)** zum Schaft **(24)** derart gewählt ist, dass die Aussenkontur des Dichtmittels, insbesondere die Aussenkontur des Dichtrings **(25)** über ein Viertel ihres Querschnittsumfangs satt anliegt und die Dichtringquerschnittsmitte **(45)** in axialer Verlängerung zur Aussenkante **(46)** des Absatzquerschnitts verläuft, damit ein in den Verschlussbuchsenbereich **(17c)** eingesetztes Pressmittel derart einpressbar ist, insbesondere eine Verschlussmutter **(47)** derart einschraubbar ist, dass eine Aufnahme überschüssiger Flüssigkeit durch eine Verformung des Dichtmittels **(25)** radial nach aussen in den Veschlussbuchsenbereich **(17c)** ohne Druckaufbau des im Hohlraum **(11, 15, 17a, 17b)** befindlichen Druckübertragungsmittels erfolgt.

**Claims**

**1.** Pressure-measuring device (1) for measurement of intra-ocular pressure, with a hollow chamber (11, 15, 17a, 17b) filled with a pressure transmission medium surrounding a compensating element (29), and said hollow chamber (11, 15, 17a, 17b) being surrounded by hollow-chamber walls, and with a pressure-measuring unit (19) which communicates with a pressure transmission membrane (27) via the pressure transmission medium, the materials and dimensions of the hollow-chamber walls, of the hollow-chamber volume ($V_{11}$, $V_{15}$, $V_{17a}$, $V_{17b}$) and of the pressure-measuring unit (19) and the nature of the pressure transmission medium being chosen such that the pressure of the pressure transmission medium at its boundary walls remains constant over a predetermined temperature range, without any external influence, and the pressure transmission membrane (27) is arranged in at least one of the walls of a hollow chamber (11), the hollow-chamber walls, the pressure transmission medium, the pressure transmission membrane (27), the compensating element (29) and the main body (3) of the device are transparent in at least one wavelength range of visible light, and the refractive index of the pressure transmission medium and subsidiary areas of the main body (3) and/or of the compensating element (29) are adapted to one another so that observations can be made through the device using an optical observation system on or in the eye.

**2.** Pressure-measuring device (1) according to Claim 1, with outer contours (5, 9) in at least two mutually opposite subsidiary areas of the device (1) which are shaped in such a way that the device (1) can be fitted into an imaging optical system.

**3.** Pressure-measuring device (1) according to Claim 1 or 2, with a liquid as pressure transmission medium.

**4.** Pressure-measuring device according to Claim 3, in which, as far as possible at each point, the hollow chamber serving for pressure transmission expands to exactly the same extent as the material combination of pressure transmission medium and compensating element located therein, so that as far as possible upon pressure transmission between pressure transmission medium (27) and pressure-measuring unit (19) a compensation, and not just a summary compensation, is achieved at each position, and an existing temperature gradient does not lead to an incorrect compensation.

**5.** Pressure-measuring device (1) according to one of Claims 1 to 4, in which the pressure transmission medium is free of gas and is preferably inert with respect to materials of the hollow-chamber walls and materials from these and/or materials diffusing through these.

**6.** Pressure-measuring device (1) according to one of Claims 1 to 5, in which the pressure-measuring unit (19) has a measurement head (20) and a shoulder (23) whose cross section is wider than the external diameter of the measurement head, and one of the hollow-chamber walls has a passage (17a, 17b, 17c) whose dimensions are chosen such that the measurement head (20) can be pushed through, and a subsidiary passage (17b) is provided whose cross section with respect to the shoulder (23) permits a lateral gap (41), the depth (t) of the subsidiary passage and the height (h) of the shoulder are chosen the same and the subsidiary passage (17b), on its side facing away from the hollow chamber, widens into a closure bushing area (17c) in such a way that a sealing means, in particular a sealing ring (25), can be fitted with play.

7. Pressure-measuring device (1) according to Claim 6, in which the shoulder (23) of the pressure-measuring unit (19) is adjoined by a shaft (24) of smaller diameter, and the contour transition (43) from the external diameter of the shoulder (23) to the shaft (24) is chosen such that the outer contour of the sealing means, in particular the outer contour of the sealing ring (25), bears closely thereon along a quarter of its cross-section circumference, and the cross-section centre (45) of the sealing ring extends in axial continuation to the outer edge (46) of the shoulder cross section so that a press means fitted into the closure bushing area (17c) can be pressed in, particularly a closure nut (47) can be screwed in, in such a way that excess liquid is taken up through a deformation of the sealing means (25) radially outwards into the closure bushing area (17c) without pressure build-up of the pressure transmission medium located in the hollow chamber (11, 15, 17a, 17b).

**Revendications**

1. Dispositif de mesure de pression (1) pour la tension oculaire, doté d'une cavité (11, 15, 17a, 17b) remplie d'un agent de transmission de pression entourant un corps d'équilibrage (29), ladite cavité étant entourée de parois, et doté d'une unité de mesure de pression (19), qui est raccordée à une membrane de transmission de pression (27) par l'intermédiaire de l'agent de transmission de pression, dans lequel les matières et les dimensions des parois de la cavité, du volume de la cavité ($V_{11}$, $V_{15}$, $V_{17a}$, $V_{17b}$) et de l'unité de mesure de pression (19), ainsi que le type d'agent de transmission de pression, sont sélectionnés de telle sorte que la pression de l'agent de transmission de pression reste constante sur ses parois de limitation, dans une plage de températures prédéfinie sans action extérieure, et la membrane de transmission de pression (27) est disposée dans au moins l'une des parois d'une cavité (11), les parois de la cavité, l'agent de transmission de pression, la membrane de transmission de pression (27), le corps d'équilibrage (29) et le corps de base (3) du dispositif sont transparents dans au moins une plage de longueurs d'ondes de la lumière visible, et l'indice de réfraction de l'agent de transmission de pression et des zones partielles du corps de base (3) et/ou du corps d'équilibrage (29) sont adaptés les uns aux autres, afin de pouvoir réaliser dans ou sur l'oeil des observations faites avec le dispositif muni d'un système d'observation optique.

2. Dispositif de mesure de pression (1) selon la revendication 1, avec des contours extérieurs (5, 9) dans au moins deux zones partielles opposées du dispositif (1), formées de telle sorte que le dispositif (1) est utilisable dans un système optique d'imagerie.

3. Dispositif de mesure de pression (1) selon la revendication 1 ou 2, avec un liquide comme agent de transmission de pression.

4. Dispositif de mesure de pression selon la revendication 3, dans lequel le plus possible en chaque endroit la cavité servant à la transmission de pression se dilate juste autant que la combinaison qui s'y trouve de matières premières de l'agent de transmission de pression et du corps d'équilibrage, afin d'obtenir le plus possible à chaque endroit, lors de la transmission de pression entre la membrane de transmission de pression (27) et l'unité de mesure de pression (19), une compensation, et une compensation pas seulement sommaire, et afin qu'un gradient de température existant entraîne une compensation erronée.

5. Dispositif de mesure de pression (1) selon l'une des revendications 1 à 4, dans lequel l'agent de transmission de pression est sans gaz et de préférence inerte par rapport aux matières des parois de la cavité ou aux matières se diffusant à partir d'elles-mêmes et/ou contre elles-mêmes.

6. Dispositif de mesure de pression (1) selon l'une des revendications 1 à 5, dans lequel l'unité de mesure (19) dispose d'une tête de mesure (20) et d'un talon (23) de section élargie par rapport au diamètre extérieur de la tête de mesure, et l'une des parois de la cavité présente une ouverture (17a, 17b, 17c), dont les dimensions sont sélectionnées de telle sorte que la tête de mesure (20) peut y être enfichée et qu'il existe une traversée partielle (17b), dont la section permet une fente latérale (41) vers le talon (23), la profondeur de la traversée partielle (t) ainsi que la hauteur du retrait (h) étant sélectionnées égales et la traversée partielle (17b) s'élargit, sur son côté tourné vers la cavité, dans une zone de douille de fermeture (17c) de telle sorte qu'un agent d'étanchéité, notamment un anneau d'étanchéité (25), peut être utilisé avec jeu.

7. Dispositif de mesure de pression (1) selon la revendication 6, dans lequel une tige (24) de diamètre diminué est adjacente au talon (23) de l'unité de mesure (19), et le passage du contour (43) allant du diamètre extérieur du retrait (23) vers la tige (24) est sélectionné de telle sorte que le contour extérieur de l'agent d'étanchéité, notamment le contour extérieur de l'anneau d'étanchéité (25), affleure sur un quart de sa périphérie de section, et le milieu de la section de l'anneau d'étanchéité (45) se termine dans le prolongement axial vers l'arête extérieure (46) de la section de retrait, afin qu'un moyen de pression inséré dans la zone de douille de fermeture (17c) peut être engagé à la presse, notamment un écrou d'obturation (47) peut être vissé, de telle sorte que le liquide en excès sort par une déformation de l'agent d'étanchéité (25) radialement vers l'extérieur dans la zone de douille de fermeture (17c), sans montée en pression de l'agent de transmission de pression se trouvant dans la cavité (11, 15, 17a, 17c).

Fig. 1

Fig. 2

Fig. 3